# EUROPEAN PATENT SPECIFICATION

(11) **EP 3 945 318 B1**
(45) Date of publication and mention of the grant of the patent: **20.12.2023**
(21) Application number: 20188976.3
(22) Date of filing: 31.07.2020
(51) Int. Cl.: G01N 33/543, C12Q 1/68

(54) **METHOD OF POOLING SAMPLES FOR ANALYTE DETECTION**
VERFAHREN ZUR BÜNDELUNG VON PROBEN FÜR ANALYTNACHWEIS
PROCÉDÉ POUR FORMER UN GROUPEMENT D'ÉCHANTILLONS POUR LA DÉTECTION D'ANALYTES

(43) Date of publication of application: 02.02.2022
(73) Proprietor: Synlab Holding Deutschland GmbH, 86156 Augsburg (DE)
(72) Inventor: Reber, Achim, 86156 Augsburg (DE)
(74) Representative: Kuttenkeuler, David

(56) References cited:
- WO-A1-99/42832
- WO-A1-2016/020455
- WO-A2-2007/075891

## Description

### FIELD OF THE INVENTION

The invention pertains to a method for detecting a pathogen derived analyte species in patient samples in accordance with the appended set of claims. Large scale diagnostic screening often uses patient sample pools to increase sample throughput. Using such patient pools, negative tests indicate that all samples in the pool are negative, whereas a positive detection leads to a deconvolution of the sample pool and individual patient sample testing to identify the one or more positive samples. The present invention uses magnetic capture particles to pool analyte from multiple samples without any substantial combination of the sample material which would otherwise lead to an unfavourable dilution of the analyte species to be detected.

### DESCRIPTION

Diagnostic assays using for example quantitative Polymerase Chain Reaction (qPCR) most commonly process patient samples one by one. While this is usually an effective and reliable method, if assay throughput is key such as in the 2020 COVID-19 pandemic, there is demand for more efficient measures. Diagnostic assays can be scaled up by the method of High-Throughput qPCR via sample pooling. Pooling, the action of combining multiple samples into one tube, is most effective when the chance of positive detection of the target analyte is low. In such cases, large groups of samples can be conclusively classified as negative with a single test of the pooled samples, with no need to individually test every sample.

However, simple pooling of sample material, such as combining the liquid samples with each other, will result in a dilution of the target analyte to be detected because in such cases it is assumed that only a minor fraction of samples within each pool does contain the target analyte species, such as a viral nucleic acid. A dilution of target analyte results in reduced assay accuracy, increased false negative rates and a limited number of samples that can be pooled while maintaining sufficient assay accuracy.

Conventional protocols for obtaining DNA or RNA from cells entail suspending the cells in a solution and using enzymes and/or chemicals, to lyse the cells, thereby releasing the nucleic acid contained within the cells into the resulting lysate solution. Glass particles, silica particles, silica gel, and mixtures of the above have been configured in various different forms to produce matrices capable of reversibly binding nucleic acid materials by adsorption to the surface when placed in contact with a medium containing such materials in the presence of binding inducing agents like chaotropic agents. Such matrices are designed to remain adsorbed to the nucleic acid material while the matrix is exposed to an external force such as centrifugation or vacuum filtration to separate the matrix and nucleic acid material adsorbed thereto from the remaining media components. The nucleic acid material usually is then eluted from the matrix by exposing the matrix to an elution solution, such as water or an elution buffer. Numerous commercial sources offer silica-based matrices designed for use in centrifugation and/or filtration isolation systems (e.g. the QiaPrep^{®},QIAamp<^{®}>and AHPrep<^{®}>lines of nucleic acid isolation systems from QIAGEN, Hilden, Germany).

Magnetically responsive particles (herein referred to as 'magnetic particles') and methods for using them have been developed for the isolation of nucleic acid materials. Several different types of magnetic particles designed for use in nucleic acid isolation are described in the literature and are available from commercial sources. Such magnetic particles generally fall into either of two categories, those designed to reversibly bind nucleic acid materials directly, and those designed to do so indirectly, i.e. through at least one intermediary substance. The intermediary substance is referred to herein as a 'label'. For example, one such commonly employed label, biotinylated oligonucleotide deoxythymidine (oligo-dT), forms hydrogen bonds with the polyadenosine tails of mRNA molecules in a medium.

WO 2007/075891 discloses methods and compositions for multiplexed assays using magnetic and non-magnetic particles. Also disclosed are methods and compositions for performing multi-step assays within a single assay vessel.

WO 2016/020455 discloses a method for automated processing of pooled samples, particularly blood samples. Furthermore, WO 2016/020455 discloses an apparatus for carrying out the method and the corresponding uses. The method, and accordingly, the apparatus, is disclosed to be used in particular to perform nucleic acid amplification techniques (NAT) for testing blood donations and blood products.

WO 99/42832 discloses a magnetic particle transfer device and method.

Thus, it is an objective of the invention to provide a method for detecting pathogen derived analyte species by pooling analyte from multiple different samples from multiple different sources, but without substantial dilution of target analyte in the pool.

### BRIEF DESCRIPTION OF THE INVENTION

Generally, and by way of brief description, the main aspects of the present invention can be described as follows:
The invention pertains to a method for detecting a pathogen derived analyte species in patient samples, the method comprising the steps of providing samples from different patients, pooling analyte of each sample with a method for pooling analyte from multiple different samples from different sources, performing a method for analyte species detection with the so pooled analyte, wherein if the analyte species is detected in the pooled analyte, another detection is performed for each patient sample individually to identify which of the one or more samples contain(s) the analyte species; and
wherein the method for pooling analyte from multiple different samples from different sources comprises the steps of:
   **(a)** Contacting a first sample with a magnetic collection device and a magnetic capture particle (or particles),
   **(b)** Binding, if not already bound, the magnetic capture particle (or particles) to the magnetic collection device by applying a magnetic force,
   **(c)** Removing the first sample from the magnetic collection device, or *vice versa,* without releasing the magnetic capture particle (or particles) from the magnetic collection device,
   **(d)** Contacting the magnetic collection device and the magnetic capture particle (or particles) with at least one further sample, wherein the at least one further sample is different from the first sample,
wherein each sample of the first sample and any of the at least one further sample(s) are distinct samples from different patients or subjects.

### .ETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.
In **a first aspect,** the invention pertains to a method for detecting a pathogen derived analyte species in patient samples, the method comprising the steps of providing samples from different patients, pooling analyte of each sample with a method for pooling analyte from multiple different samples from different sources, performing a method for analyte species detection with the so pooled analyte, wherein if the analyte species is detected in the pooled analyte, another detection is performed for each patient sample individually to identify which of the one or more samples contain(s) the analyte species; and
wherein the method for pooling analyte from multiple different samples from different sources comprises the steps of:
   (a) Contacting a first sample with a magnetic collection device and a magnetic capture particle (or particles),
   (b) Binding, if not already bound, the magnetic capture particle (or particles) to the magnetic collection device by applying a magnetic force,
   (c) Removing the first sample from the magnetic collection device, or *vice versa,* without releasing the magnetic capture particle (or particles) from the magnetic collection device,
   (d) Contacting the magnetic collection device and the magnetic capture particle (or particles) with at least one further sample, wherein the at least one further sample is different from the first sample,
wherein each sample of the first sample and any of the at least one further sample(s) are distinct samples from different patients or subjects.

The term "magnetic particle" or "magnetic capture particle" refers to a particle which can be attracted by a magnetic field. In the present invention, the magnetic particle preferably has an average diameter of at most 1000 µm, in particular of at least 0.1 µm to less than 1000 µm, also referenced as magnetic microparticle (MMP). Preferably, the magnetic particle has an average diameter of from about 0.5 µm to about 100 µm. The magnetic particle may have an average diameter of about 0.5 µm to 20 µm, preferably about 0.6 µm to about 10 µm, more preferably about 0.8 µm to about 5 µm. The magnetic particle can be obtained from commercial suppliers and the ordinary of skill in the art is also aware of methods for preparing it.

Preferably, the magnetic capture particle comprises magnetically responsive particles which are able to reversibly bind nucleic acids in the terms of the present invention, e.g. by ionic interaction or the like. Such particles are well known to a person skilled in the art. As used herein, the term 'magnetic' encompasses magnetic materials, such as ferromagnetic, ferrimagnetic, paramagnetic or superparamagnetic materials. These magnetic materials are part of the magnetic particles as described above and may be included in the particles by any suitable method.

In a preferred embodiment of the invention, the magnetic particles comprise silica. The silica could be in the form of silica gel, siliceous oxide, solid silica such as glass or diatomaceous earth, or a mixture of two or more of the above. The silica should be present at least in part on the surface of the beads. For efficient binding, it is preferred that the silica is present on the entire surface of the particles.

The magnetic particles may be silica coated magnetic particles. The term 'silica coated magnetic particles' refers to magnetic particles coated with silica. These particles are well known to the artisan. Any kind of silica coated magnetic particles is in principle suitable for the method of the present invention. Yet, preferably the magnetic particles are no anion exchange particles.

The glass particles are preferably particles of crystalline silica (e.g., a-quartz, vitreous silica), even though crystalline silica is not formally 'glass' because it is not amorphous, or particles of glass made primarily of silica. Glass particles are also well known to the skilled person.

Siliceous-oxide coated magnetic particles are the most preferred form of silica magnetic particles used in the present invention. Siliceous-oxide coated magnetic particles are comprised of siliceous oxide coating a core comprising at least one particle of ferrimagnetic, ferromagnetic, superparamagnetic or paramagnetic material. The siliceous-oxide coated magnetic particles used in the present invention also have an adsorptive surface of hydrous siliceous oxide. The target nucleic acid, such as DNA or RNA, adheres to the adsorptive surface of the particles while other material from the source of the nucleic acid, particularly deleterious contaminants such as nucleases or the like, do not adhere to or co-elute from the siliceous-oxide coated magnetic particles together with the nucleic acid. Siliceous-oxide coated magnetic particles are well known to the artisan and are commercially available (e.g. MagAttract^{®} magnetic particles, QIAGEN, Hilden, Germany).

The magnetic particles have the capacity to form a complex with an analyte molecule of the invention, such as a nucleic acid, in the aqueous solution by reversibly binding the analyte, such as the nucleic acid, e.g. by ionic interaction or the like. The present invention may be performed using any silica coated magnetic particles possessing the property of forming a complex with the nucleic acid.

The term "magnetic collection device" is used herein to describe a magnet; such as a magnetizable rod-shaped magnet; or a magnetizable plate with one or more receiving compartments (such as a well plate) to receive a sample liquid, alternatively a well plate which is placed on top of a magnet.

The magnet can be of any material, such as a high performance magnet. The magnet can be a rare earth magnet or a magnet selected from among an R-cobalt magnet and an R-Fe-B magnet, wherein R is selected from among lanthanum (La), cerium (Ce), praseodymium (Pr), neodymium (Nd), samarium (Sm), europium (Eu), gadolinium (Gd), terbium (Tb), dysprosium (Dy), holmium (Ho), erbium (Er), thulium (Tm), ytterbium (Yb), lutetium (Lu)), scandium (Sc) and yttrium (Y). The magnet can be a neodymium magnet that includes neodymium, iron, and boron, such as an alloy of the composition Nd2Fe14B. The magnet can be a samarium-cobalt magnet, such as a magnet having a composition selected from among SmCO5 and Sm2CO17.

Preferably, the magnetic collection device is a magnetic rod which can be introduced into a sample liquid in order to contact the magnetic rod and the sample and the magnetic capture particle (or particles); or wherein the magnetic collection device is a magnetic compartment which can hold a sample liquid in order to contact the magnetic compartment and the sample liquid and the magnetic capture particle (or particles).

The term "analyte molecule" is used herein to refer to a molecule or a part thereof of which a specific species is the target of the detection of the present invention. Analyte molecule is subject of the detection and pooling methods according to the embodiments of the present invention. Analyte molecules include biological molecules (such as, but not limited to, proteins, peptides, lipids, nucleic acids, fatty acids or carbohydrates, such as oligosaccharides) and nonbiological molecules (including small molecules, such a small molecule drugs or small molecule ligands). Nucleic acids are preferred analyte molecules of the invention.

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. Nucleic acids include, but are not limited to all types of DNA and/or RNA, e.g. gDNA; plasmid DNA, circular DNA; circulating DNA; hnRNA; mRNA; noncoding RNA (ncRNA), including but not limited to rRNA, tRNA, IncRNA (long non coding RNA), lincRNA (long intergenic non coding RNA), miRNA (micro RNA), siRNA (small interfering RNA), snoRNA (small nucleolar RNA), snRNA (small nuclear RNA) and stRNA (small temporal RNA), piRNA (piwi interacting RNA), tiRNA (transcription initiation RNA), PASR (promoter associated RNA), CUT (cryptic unstable transcripts), extracellular or circulating RNA; fragmented nucleic acid; nucleic acid obtained from subcellular organelles such as mitochondria or chloroplasts; and nucleic acid obtained from microorganisms, parasites, or DNA or RNA viruses that may be present in a biological sample. Synthetic nucleic acid sequences that may or may not include nucleotide analogues that are added or "spiked" into a biological sample are also within the scope of the invention. Small RNA or the term small RNA species in particular refers to RNA having a length of less than 500nt. According to one embodiment, the nucleic acid is DNA. A sample may comprise more than one type of nucleic acid. Depending on the intended use, it may be desirous to isolate all types of nucleic acids from a sample ((e.g. DNA and RNA) or only certain types or a certain type of nucleic acid (e.g. only RNA but not DNA or vice versa or DNA and RNA are supposed to be obtained separately). All these variants are within the scope of the present invention. Suitable methods for isolating either DNA or RNA or both types of nucleic acids in parallel are known in the prior art.

The term "sample" is used herein in a broad sense and is intended to include a variety of sources that contain nucleic acids. The sample may be a biological sample but the term also includes other, e.g. artificial samples which comprise nucleic acids. Exemplary samples include, but are not limited to, body fluids in general, whole blood; serum; plasma; red blood cells; white blood cells; buffy coat; swabs, including but not limited to buccal swabs, throat swabs, vaginal swabs, urethral swabs, cervical swabs, throat swabs, rectal swabs, lesion swabs, abcess swabs, nasopharyngeal swabs, and the like; urine; sputum; saliva; semen; lymphatic fluid; liquor; amniotic fluid; cerebrospinal fluid; peritoneal effusions; pleural effusions; fluid from cysts; synovial fluid; vitreous humor; aqueous humor; bursa fluid; eye washes; eye aspirates; plasma; serum; pulmonary lavage; lung aspirates; and tissues, including but not limited to, liver, spleen, kidney, lung, intestine, brain, heart, muscle, pancreas, cell cultures, as well as lysates, extracts, or materials obtained from any cells and microorganisms and viruses that may be present on or in a sample and the like. Materials obtained from clinical or forensic settings that contain nucleic acids are also within the intended meaning of the term sample. Furthermore, the skilled artisan will appreciate that lysates, extracts, or materials or portions thereof obtained from any of the above exemplary samples are also within the scope of the term sample. Preferably, the sample is a biological sample derived from a human, animal, plant, bacteria or fungi. In particular, the term "sample" refers to a nucleic acid containing sample which also comprises proteins. Preferably, the sample is selected from the group consisting of cells, tissue, bacteria, virus and body fluids such as for example blood, blood products such as buffy coat, plasma and serum, urine, liquor, sputum, stool, CSF and sperm, epithelial swabs, biopsies, bone marrow samples and tissue samples, preferably organ tissue samples such as lung and liver. Preferably, the sample is selected from whole blood and blood products such as buffy coat, serum or plasma. The nucleic acid may also be an artificially synthesized nucleic acid, for example from a PCR reaction. The sample solution used in the process could be obtained directly from the biological material, for example by lysis of cells or tissue or by fractionation and/or pretreatment of body liquids. Alternatively, the sample solution could be obtained from a nucleic acid processing method, which may be a method for purification, synthesis or modification, such as PCR or purification from agarose gel.

Hence, the method of the present invention can be realized in an embodiment of steps a to d in which one single set of magnetic capture particles is brought into contact with multiple samples to collect and thereby pool analyte bound the magnetic particles. The method of the invention avoids a step of pooling sample material directly which would result in an undesired dilution of the target analyte in a potential positive sample pool.

In one preferred embodiment of the invention the at least one further sample comprises at least two, three, four, five, six, seven, eight, nine or ten, or 11 to 15, or 11 to 20, or 20 to 50, 100, 150 or more individual samples. One advantage of the method of the invention is that due to avoiding a dilution or reduction of the concentration of target analyte, more samples can be combined within one pool compared to prior art methods.

As mentioned before, the method of the invention preferably does not comprise a direct and/or substantial combination of liquids of any two of the first and the at least one further sample.

Furthermore, the method of the invention does not require, and hence preferably does not include, any additional step of concentrating analyte molecules, for example using any filters, centrifugation (for example after precipitation), or binding columns.

In one particularly preferred embodiment of the invention, the step of contacting the first or the at least one further sample with the magnetic capture particle (or particles) or the at least one further magnetic capture particle (or particles), respectively, comprises a contacting for a time sufficient to capture potentially present analyte in the sample to the magnetic capture particle (or particles). Usually, the term "contacting" means brining two or more entities in close spatial proximity such they can interact with each other, for example, for the magnetic collection device and the magnetic capture particles, they are in such spatial proximity that if the magnetic collection device is magnetized, the magnetic capture particles can move within the magnetic field and attach to the magnetic collection devices surface.

Preferably each sample of the first sample and any of the at least one further sample(s) are distinct from each other, preferably wherein each of said samples can independently comprise a target analyte or not. The term "target analyte" shall refer to a species of analyte molecules which is subject of the detection method, such as a viral nucleic acid to be detected in a biological sample which usually comprises many other non-target nucleic acids, such as nucleic acids of the host. Distinct samples in context of the invention are preferably samples from different patients or subjects.

Yet another embodiment of the invention requires that the analyte is a molecular compound capable to specifically bind the magnetic capture particle (or particles), preferably wherein the analyte is a biological molecule such as a nucleic acid, peptide, protein, lipid, carbohydrate (sugar) or any combination thereof. For silica particles for example it is known that they can bind nucleic acids. However, other analyte might require different particle materials or coating, for example antibodies for capturing certain proteins or the like.

In some embodiments of the invention, between steps (c) and (d) the magnetic capture particle (or particles) is (are) washed. Such washing steps may be repeated multiple times.

In some embodiments the magnetic capture particle (or particles) is contacted with the analyte within each sample by releasing the magnetic capture particle (or particles) from the magnetic capture device into the sample liquid. Such releasing may be performed by demagnetizing the magnetic collection device, optionally with an additional stirring of the device in the sample liquid. Alternatively other means or removing the particles, using further magnets or devices for separation known to the skilled artisan shall be comprised in the scope of the invention.

In further embodiments step (a) and/or (d) comprises a step of stirring the sample liquid.

Performed according to the description the method of the invention results in combining and/or admixing two or more magnetic capture particles each contacted with different samples, and each potentially are bound to analyte from different samples.

Furthermore, the method may comprise a step of removing from the one or more magnetic capture particle(s) any potentially bound analyte. Subsequently the eluted analyte may be subject to a detection method, such as a PCR based method, for determining the presence of absence of target analyte in the eluted pool of analyte.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

### BRIEF DESCRIPTION OF THE FIGURES

The figures show:
**Figure 1****:** shows a setup of a prior art sample handling.
**Figure 2****:** shows a setup of a first embodiment of the invention.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Comparative Example 1: Prior Art Nucleic Acid Isolation

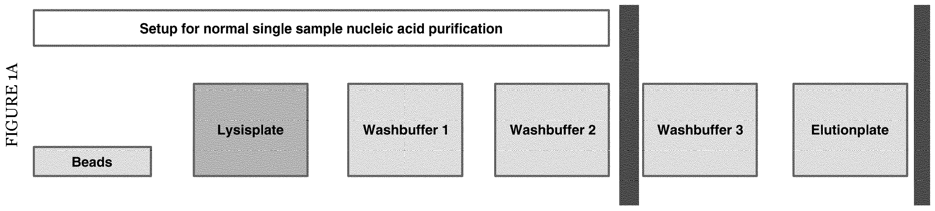

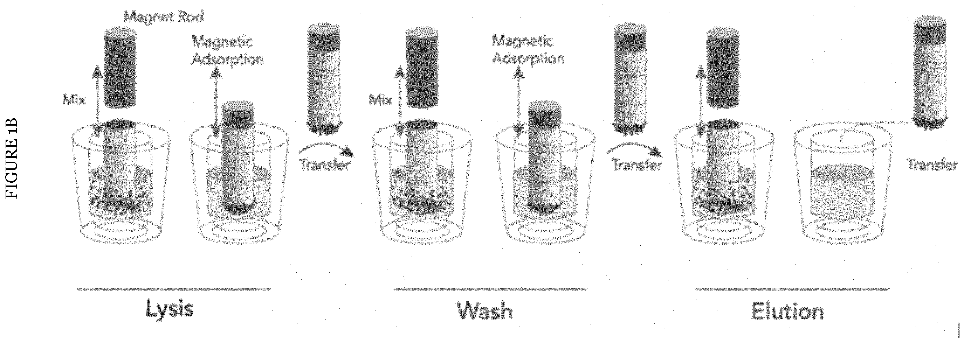

Figure 1A and Figure 1B show a prior art method for capturing nucleic acids using magnetic capture beads. Lysates of one individual sample or of pooled samples is admixed with magnetic beads to bind analyte nucleic acids using a magnet. The beads are then transferred with the magnet to a washing solution and thereafter eluted for final analysis.

### Example 1:

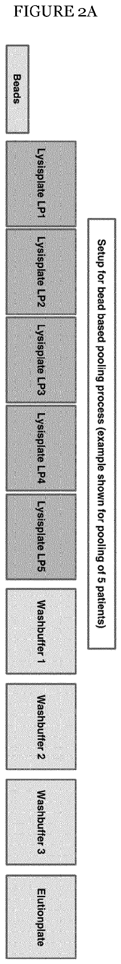

Figure 2A shows a typical plate layout for performing a preferred embodiment of the method of the invention. Each lysis plate comprises lysates of individual samples (patient samples) to be pooled according to the method of the invention. If the plate comprises multiple wells, multiple pooling procedures are performed in parallel. Then the samples of one plate coordinate are pooled with each other. The number of plates indicates pool size, which can be customized by using 3 lysis plates for pooling of 3 patients, 7 lysis plates for pooling of 7 patients and so on. The pooling process is done by collecting nucleic acids from position A1 of LP1, A1 (LP2), A1 (LP3), A1 (LP4) and A1 (LP5) successively. Using a 96 rod head, all wells are pooled in parallel. Other formats are possible (348, 12, 6 etc.)

The general setup of the nucleic acid purification (beads, wash buffers and elution plate) may vary depending on the preparation chemistry used. The setup shown is V300 preparation from Chemagen^{®}. For the bead based pooling process other extraction kits from different companies can be used also, the basic condition needed is only, that magnetic beads can be transported from one plate to the next plate. Therefore systems like the King Fisher Flex ^{®}, the IDEAL^{®} 96 extraction robot or the Biosprint^{®} 96 can be used also.

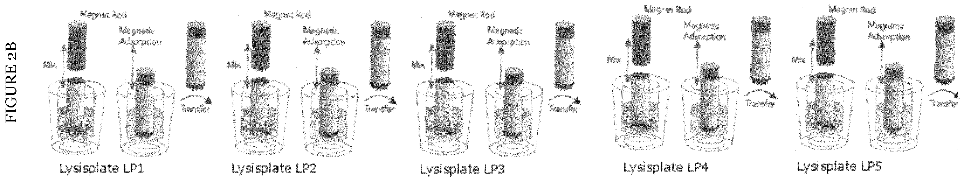

As shown in Figure 2B, the magnetic rod head is then sequentially brought into contact with each sample plate, possible washing steps as in the prior art method are possible. Pooling process during sample lysis is achieved by one set of beads which is transferred through all lysis plates in order to collect the nucleic acids from corresponding wells. Afterwards washing and elution steps are performed as normal.

### Comparative Example 2:

In example 2, instead of transferring the particles to each lysis plate, the particles are added to a magnetic plate for collection, and multiple lysates are sequentially added to the plate holding the particles. After incubation the particles are attached to the plate bottom and the lysates are aspirated, optionally the plate is washed, and then contacted with a further sample to pool analyte. After pooling process, the extraction continues with normal washing and elution steps.

## Claims

1. **A method for detecting a pathogen derived analyte species in patient samples,** the method comprising the steps of providing samples from different patients, pooling analyte of each sample with a method for pooling analyte from multiple different samples from different sources, performing a method for analyte species detection with the so pooled analyte, wherein if the analyte species is detected in the pooled analyte, another detection is performed for each patient sample individually to identify which of the one or more samples contain(s) the analyte species; and
wherein the method for pooling analyte from multiple different samples from different sources comprises the steps of:
**(a)** Contacting a first sample with a magnetic collection device and a magnetic capture particle (or particles),
**(b)** Binding, if not already bound, the magnetic capture particle (or particles) to the magnetic collection device by applying a magnetic force,
**(c)** Removing the first sample from the magnetic collection device, or *vice versa,* without releasing the magnetic capture particle (or particles) from the magnetic collection device,
**(d)** Contacting the magnetic collection device and the magnetic capture particle (or particles) with at least one further sample, wherein the at least one further sample is different from the first sample,
wherein each sample of the first sample and any of the at least one further sample(s) are distinct samples from different patients or subjects.

2. The method according to claim 1, wherein the at least one further sample comprises at least two, three, four, five, six, seven, eight, nine or ten, or 11 to 15, or 11 to 20, or 20 to 50, 100, 150 or more individual samples.

3. The method of claim 1 or 2, wherein the method does not comprise a direct and/or substantial combination of liquids of any two of the first and the at least one further sample.

4. The method of any one of claims 1 to 3, wherein the step of contacting the first or the at least one further sample with the magnetic capture particle (or particles) or the at least one further magnetic capture particle (or particles), respectively, comprises a contacting for a time sufficient to capture potentially present analyte in the sample to the magnetic capture particle (or particles).

5. The method of any one of claims 1 to 4, wherein the samples are biological samples.

6. The method of any one of claims 1 to 5, wherein each of said samples can independently comprise an analyte or not.

7. The method of any one of claims 1 to 6, wherein the analyte a molecular compound capable to specifically bind the magnetic capture particle (or particles), preferably wherein the analyte is a biological molecule such as a nucleic acid, peptide, protein, lipid, carbohydrate (sugar) or any combination thereof.

8. The method of any one of claims 1 to 7, wherein the magnetic collection device is a magnetic rod which can be introduced into a sample liquid in order to contact the magnetic rod and the sample and the magnetic capture particle (or particles); or wherein the magnetic collection device is a magnetic compartment which can hold a sample liquid in order to contact the magnetic compartment and the sample liquid and the magnetic capture particle (or particles).

9. The method of any one of claims 1 to 8, wherein between steps (c) and (d) the magnetic capture particle (or particles) is washed.

10. The method of any one of claims 1 to 9, wherein each sample is a liquid sample, and said liquid comprises the analyte.

11. The method of any one of claims 1 to 10, wherein the magnetic capture particle (or particles) is contacted with the analyte within each sample by releasing the magnetic capture particle (or particles) from the magnetic capture device into the sample liquid.

12. The method of any one of claims 1 to 11, wherein step (a) and/or (d) comprises a step of stirring the sample liquid.

## Patentansprüche

1. Verfahren zum Nachweis einer Pathogen-abgeleiteten Analytspezies in Patientenproben, das Verfahren umfassend die Schritte eines Bereitstellens von Proben von verschiedenen Patienten, eines Zusammenfassens von Analyt jeder Probe mit einem Verfahren zum Zusammenfassen von Analyt von einer Vielzahl von verschiedenen Proben von verschiedenen Quellen, eines Durchführens eines Verfahrens für einen Analytspeziesnachweis mit dem so zusammengefassten Analyten, wobei, falls die Analytspezies in dem zusammengefassten Analyten nachgewiesen wird, ein anderer Nachweis für jede Patientenprobe einzeln durchgeführt wird, um zu identifizieren, welche der einen oder der mehreren Proben die Analytspezies enthält/enthalten; und
wobei das Verfahren zum Zusammenfassen von Analyt von der Vielzahl von verschiedenen Proben von verschiedenen Quellen die Schritte umfasst:
(a) Inberührungbringen einer ersten Probe mit einer magnetischen Sammelvorrichtung und einem magnetischen Fängerpartikel (oder -partikeln),
(b) Binden, falls nicht bereits gebunden, des magnetischen Fängerpartikels (oder der -partikel) an die magnetische Sammelvorrichtung durch Anwenden einer magnetischen Kraft,
(c) Entfernen der ersten Probe von der magnetischen Sammelvorrichtung, oder *umgekehrt,* ohne den magnetischen Fängerpartikel (oder die -partikel) von der magnetischen Sammelvorrichtung freizusetzen,
(d) Inberührungbringen der magnetischen Sammelvorrichtung und des magnetischen Fängerpartikels (oder der -partikel) mit mindestens einer weiteren Probe, wobei die mindestens eine weitere Probe von der ersten Probe verschieden ist,
wobei jede Probe der ersten Probe und eine beliebige der mindestens einen weiteren Probe(n) unterschiedliche Proben von verschiedenen Patienten oder Subjekten sind.

2. Verfahren nach Anspruch 1, wobei die mindestens eine weitere Probe mindestens zwei, drei, vier, fünf, sechs, sieben, acht, neun oder zehn oder 11 bis 15 oder 11 bis 20 oder 20 bis 50, 100, 150 oder mehr einzelne Proben umfasst.

3. Verfahren nach Anspruch 1 oder 2, wobei das Verfahren keine direkte und/oder wesentliche Kombination von Flüssigkeiten von beliebigen zwei der ersten und der mindestens einen weiteren Probe umfasst.

4. Verfahren nach einem der Ansprüche 1 bis 3, wobei der Schritt des Inberührungbringens der ersten oder der mindestens einen weiteren Probe mit dem magnetischen Fängerpartikel (oder den -partikeln) beziehungsweise dem mindestens einen weiteren magnetischen Fängerpartikel (oder den -partikeln) ein Inberührungbringen, für eine Zeit, die ausreichend ist, um potenziell vorhandenen Analyt in der Probe einzufangen, mit dem magnetischen Fängerpartikel (oder den -partikeln) umfasst.

5. Verfahren nach einem der Ansprüche 1 bis 4, wobei die Proben biologische Proben sind.

6. Verfahren nach einem der Ansprüche 1 bis 5, wobei jede der Proben unabhängig voneinander einen Analyten umfassen kann oder nicht.

7. Verfahren nach einem der Ansprüche 1 bis 6, wobei der Analyt eine Molekülverbindung, die in der Lage ist, den magnetischen Fängerpartikel (oder die -partikel) spezifisch zu binden, vorzugsweise wobei der Analyt ein biologisches Molekül, wie eine Nukleinsäure, ein Peptid, ein Protein, ein Lipid, ein Kohlenhydrat (Zucker) oder eine beliebige Kombination davon ist.

8. Verfahren nach einem der Ansprüche 1 bis 7, wobei die magnetische Sammelvorrichtung ein magnetischer Stab ist, der in eine Probenflüssigkeit eingeführt werden kann, um den magnetischen Stab und die Probe und den magnetischen Fängerpartikel (oder die -partikel) in Berührung zu bringen; oder wobei die magnetische Sammelvorrichtung eine magnetische Kammer ist, die eine Probenflüssigkeit fassen kann, um die magnetische Kammer und die Probenflüssigkeit und den magnetischen Fängerpartikel (oder die -partikel) in Berührung zu bringen.

9. Verfahren nach einem der Ansprüche 1 bis 8, wobei zwischen den Schritten (c) und (d) der magnetische Fängerpartikel (oder die -partikel) gewaschen wird.

10. Verfahren nach einem der Ansprüche 1 bis 9, wobei jede Probe eine Flüssigkeitsprobe ist und die Flüssigkeit den Analyten umfasst.

11. Verfahren nach einem der Ansprüche 1 bis 10, wobei der magnetische Fängerpartikel (oder die -partikel) mit dem Analyten innerhalb jeder Probe durch Freisetzen des magnetischen Fängerpartikels (oder der -partikel) von der magnetischen Fängervorrichtung in die Probenflüssigkeit in Berührung gebracht wird.

12. Verfahren nach einem der Ansprüche 1 bis 11, wobei der Schritt (a) und/oder (d) einen Schritt eines Rührens der Probenflüssigkeit umfasst.

## Revendications

1. **Procédé de détection d'une espèce d'analyte dérivée d'un pathogène dans des échantillons de patients,** le procédé comprenant les étapes consistant à fournir des échantillons à partir de patients différents, à regrouper l'analyte de chaque échantillon par un procédé de regroupement d'un analyte à partir de multiples échantillons différents de sources différentes, à mettre en oeuvre un procédé de détection d'espèces d'analyte avec l'analyte ainsi regroupé, dans lequel, si l'espèce d'analyte est détectée dans l'analyte regroupé, une autre détection est effectuée pour chaque échantillon de patient individuellement afin d'identifier lequel des un ou plusieurs échantillons contiennent les espèces d'analyte ; et
dans lequel le procédé de regroupement d'un analyte provenant de multiples échantillons différents de sources différentes comprend les étapes consistant à :
(a) Mettre en contact un premier échantillon avec un dispositif de collecte magnétique et une particule (ou des particules) de capture magnétique,
(b) Relier, si ce n'est déjà le cas, la particule (ou les particules) de capture magnétique au dispositif de collecte magnétique par application d'une force magnétique,
(c) Extraire le premier échantillon du dispositif de collecte magnétique, ou *vice versa* sans libérer la particule (ou les particules) de capture magnétique du dispositif de collecte magnétique,
(d) Mettre en contact le dispositif de collecte magnétique et la particule (ou les particules) de capture magnétique avec au moins un échantillon supplémentaire, dans lequel l'au moins un échantillon supplémentaire est différent du premier échantillon,
dans lequel chaque échantillon du premier échantillon et l'un quelconque de l'au moins un échantillon supplémentaire sont des échantillons distincts provenant de patients ou de sujets différents.

2. Procédé selon la revendication 1, dans lequel l'au moins un échantillon supplémentaire comprend au moins deux, trois, quatre, cinq, six, sept, huit, neuf ou dix, ou 11 à 15, ou 11 à 20, ou 20 à 50, 100, 150 échantillons individuels ou plus.

3. Procédé selon la revendication 1 ou 2, dans lequel le procédé ne comprend pas une combinaison directe et/ou substantielle de liquides de deux quelconques du premier et de l'au moins un échantillon supplémentaire.

4. Procédé selon l'une quelconque des revendications 1 à 3, dans lequel l'étape de mise en contact du premier ou de l'au moins un échantillon supplémentaire avec la particule (ou les particules) de capture magnétique ou l'au moins une autre particule (ou d'autres particules) de capture magnétique, respectivement, comprend une mise en contact pendant une durée suffisante pour capturer un analyte potentiellement présent dans l'échantillon à la particule (ou aux particules) de capture magnétique.

5. Procédé selon l'une quelconque des revendications 1 à 4, dans lequel les échantillons sont des échantillons biologiques.

6. Procédé selon l'une quelconque des revendications 1 à 5, dans lequel chacun desdits échantillons peut comprendre indépendamment un analyte ou non.

7. Procédé selon l'une quelconque des revendications 1 à 6, dans lequel l'analyte est un composé moléculaire capable de lier spécifiquement la particule (ou les particules) de capture magnétique, de préférence dans lequel l'analyte est une molécule biologique telle qu'un acide nucléique, un peptide, une protéine, un lipide, un glucide (sucre) ou une quelconque combinaison de ceux-ci.

8. Procédé selon l'une quelconque des revendications 1 à 7, dans lequel le dispositif de collecte magnétique est une tige magnétique qui peut être introduite dans un liquide échantillon afin de venir en contact avec la tige magnétique et l'échantillon et la particule (ou les particules) de capture magnétique ; ou dans lequel le dispositif de collecte magnétique est un compartiment magnétique qui peut contenir un échantillon liquide afin de venir en contact avec le compartiment magnétique et l'échantillon liquide et la particule (ou les particules) de capture magnétique.

9. Procédé selon l'une quelconque des revendications 1 à 8, dans lequel, entre les étapes (c) et (d), la particule (ou les particules) de capture magnétique est (sont) lavée(s).

10. Procédé selon l'une quelconque des revendications 1 à 9, dans lequel chaque échantillon est un échantillon liquide, et ledit liquide comprend l'analyte.

11. Procédé selon l'une quelconque des revendications 1 à 10, dans lequel la particule (ou les particules) de capture magnétique est (sont) mise(s) en contact avec l'analyte au sein de chaque échantillon en libérant la particule (ou les particules) de capture magnétique du dispositif de capture magnétique dans le liquide échantillon.

12. Procédé selon l'une quelconque des revendications 1 à 11, dans lequel l'étape (a) et/ou (d) comprend une étape d'agitation de l'échantillon liquide.
